# EUROPÄISCHE PATENTANMELDUNG

(11) **EP 2 044 859 A1**
(43) Veröffentlichungstag der Anmeldung: **08.04.2009**
(21) Anmeldenummer: 08015808.2
(22) Anmeldetag: 09.09.2008
(51) Int. Cl.: A45D 19/00, A61Q 5/10, B05B 7/26, A45D 34/04

(54) **Haarfärbeverfahren**

(30) Priorität: 01.10.2007 DE 102007047102
(71) Anmelder: Henkel AG & Co. KGaA, 40589 Düsselsdorf (DE)
(72) Erfinder: Hoepfner, Stefan, 22523 Hamburg (DE); Kleen, Astrid, 20457 Hamburg (DE)
(74) Vertreter: Strohe-Kamp, Geertje

(57) **Zusammenfassung**

Verfahren zur Erzeugung graphischer Muster auf menschlichem Haar, wobei ein Haarfärbemittel, enthaltend mindestens einen direktziehenden Farbstoff in einem kosmetisch akzeptablen Träger in dem gewünschten Muster auf das zu behandelnde Haar aufgesprüht wird.

## Beschreibung

Die vorliegende Erfindung betrifft ein Verfahren zur Erzeugung graphischer Muster auf menschlichem Haar, wobei ein Haarfärbemittel, enthaltend mindestens einen direktziehenden Farbstoff, auf das zu behandelnde Haar aufgesprüht wird.

Die Färbung menschlicher Haare ist heute weit verbreitet. Ziel einer entsprechenden Färbung ist in der Regel, dem Kopfhaar einheitlich die gewünschte Farbe zu verleihen. In manchen Fällen sollen jedoch nur bestimmte Bereiche des Haars eingefärbt werden, um etwa Strähnchen oder sogar komplexere graphische Muster zu erzielen.

Zur permanenten Färbung menschlicher Haare kommen Haarfärbemittel zum Einsatz, die auf sogenannten Oxidationsfarbstoffvorprodukten basieren. Dabei handelt es sich um farblose organische Moleküle, die erst unter Einfluss eines Oxidationsmittels einen Farbstoff ausbilden. Wird keine permanente, sondern eine demi- oder semipermanente Färbung gewünscht, kommen als farbverändernde Substanzen so genannte direktziehende Farbstoffe zum Einsatz. Dabei handelt sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Die erzielten Färbungen überstehen eine gewisse Anzahl von Haarwäschen, wobei je nach Wahl der Farbstoffe eine Haltbarkeit der Färbung bis zu 15 Haarwäschen und darüber erreicht werden kann, werden letztlich jedoch aus dem Haar ausgewaschen.

Herkömmliche Haarfärbemittel werden in der Regel als Creme, Gel, Lotion oder auch als Schaumprodukt konfektioniert und mittels eines Pinsels oder der Hände ins Haar eingearbeitet. Soll nicht das gesamte Haar, sondern nur bestimmte Partien eingefärbt werden, sind diese vor dem Färbevorgang aufwändig vom restlichen Haar zu separieren.

Zum Auftragen komplexer graphischer Muster auf das Haar schlägt WO 99/59443 A1 die Verwendung verschiedener Schablonen vor. Zur Erzielung eines Musters kommen demnach mehrere Schablonen zum Einsatz. Es wird eine erste Schablone auf dem Haar fixiert. Durch die Öffnungen der Schablone wird das Färbemittel mit einem Pinsel auf das Haar aufgebracht. Anschließend wird eine weitere Schablone fixiert und erneut durch die Öffnungen Färbemittel aufgebracht. Dieser Vorgang wird wiederholt, bis das gewünschte Muster entstanden ist. Obwohl sich mittels dieses Vorgehens sehr komplexe Muster erzielen lassen, weist der Prozess eine Reihe von Nachteilen auf. Durch den zwingenden Einsatz verschiedener Schablonen wird der Färbevorgang sehr arbeits- und zeitaufwändig. Zudem ist das Vorgehen wenig flexibel. Für jedes gewünschte Muster muss ein separater Satz an Schablonen hergestellt werden. Der Kreativität des Friseurs oder sonstigen Anwenders sind damit enge Grenzen gesetzt.

Aus dem Bereich des Bodypainting kennt man das Aufsprühen von Färbemitteln direkt auf die Haut. Dabei kommen pigmenthaltige Färbemittel zum Einsatz, die sich durch einfaches Abwaschen wieder rückstandsfrei entfernen lassen. Sprayförmige Mittel zum Färben der Haare sind auf dem Gebiet des Karnevalszubehörs bekannt. Auch hier handelt es sich um Färbemittel auf Basis von Pigmenten, insbesondere anorganischen Pigmenten, die schon bei einmaliger Haarwäsche wieder aus dem Haar ausgespült werden. Mittels solcher sprayförmiger Färbemittel lassen sich demnach keine beständigen farbigen Muster auf dem Haar erzielen.

Aufgabe der vorliegenden Erfindung war es daher, ein Verfahren zur Erzeugung graphischer Muster auf menschlichem Haar bereitzustellen, das es einerseits erlaubt, auf einfache Weise eine Vielzahl beliebig gestalteter Muster zu verwiklichen, wobei andererseits die Muster gegenüber mehrmaliger Haarwäsche beständig sind.

Es wurde nunmehr gefunden, dass dies durch das Aufsprühen von Haarfärbemitteln erreicht werden kann, die mindestens einen direktziehenden Farbstoff enthalten.

Gegenstand der vorliegenden Erfindung ist daher ein Verfahren zur Erzeugung graphischer Muster auf menschlichem Haar, dadurch gekennzeichnet, dass ein Haarfärbemittel, enthaltend mindestens einen direktziehenden Farbstoff in einem kosmetisch akzeptablen Träger in dem gewünschten Muster auf das zu behandelnde Haar aufgesprüht wird.

Unter dem Begriff graphisches Muster werden dabei sowohl einfache Formen, wie Punkte, Linien, Strähnen, als auch komplexe Formen bis hin zu ganzen Bildern verstanden.

Die Haarfärbemittel, die im erfindungsgemäßen Verfahren zum Einsatz kommen, enthalten mindestens einen direktziehenden Farbstoff. Im Gegensatz zu anorganischen Pigmenten erlauben diese Farbstoffe Färbeergebnisse, die gegen mehrmaliges Waschen der Haare beständig sind.

Vorzugsweise werden direktziehende Farbstoffe eingesetzt, die ausgewählt sind aus Nitrophenylendiaminen, Nitroaminophenolen, Azofarbstoffen, Anthrachinonen und Indophenolen.

Die direktziehenden Farbstoffe werden jeweils bevorzugt in einer Menge von 0,001 bis 20 Gew.-%, bezogen auf die gesamte Anwendungszubereitung, eingesetzt, besonders bevorzugt in einer Menge von 0,005 bis 10 Gew.-%, ganz besonders bevorzugt in einer Menge von 0,01 bis 5 Gew.-%. Die Gesamtmenge an direktziehenden Farbstoffen beträgt vorzugsweise höchstens 20 Gew.-%. Die Mengenangaben beziehen sich dabei auf die Anwendungszubereitung, d.h. das Haarfärbemittel, wobei gegebenenfalls im Haarfärbemittel enthaltene Treibmittel nicht berücksichtigt werden.

Direktziehende Farbstoffe können in anionische, kationische und nichtionische direktziehende Farbstoffe unterteilt werden.

Anionische direktziehende Farbstoffe:
Als anionische direktziehende Farbstoffe eignen sich insbesondere 6-Hydroxy-5-[(4-sulfophenyl)azo]-2-naphthalinsulfonsäuredinatriumsalz (C.I. 15,985; Food Yellow No. 3; FD&C Yellow No. 6), 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 4-((4-Amino-3-sulfophenyl)azo)benzolsulfonsäure-dinatriumsalz (C.I. 13,015, Acid Yellow 9), 5-Hydroxy-1-(4-sulfophenyl)-4-[(4-sulfophenyl)azo]pyrazol-3-carbonsäure-trinatriumsalz (C.I. 19,140; Food Yellow No. 4; Acid Yellow 23), 3-[(4-Phenylamino)phenyl]azobezolsulfonsäuresäure-natriumsalz (C.I. 13,065; Ki406; Acid Yellow 36), 9-(2-Carboxyphenyl)-6-hydroxy-3H-xanthen-3-on (C.I. 45,350; Acid Yellow 73; D&C Yellow No. 8), 5-[(2,4-Dinitrophenyl)amino]-2-phenylaminobenzolsulfonsäure-natriumsalz (C.I. 10,385; Acid Orange 3), 4-[(2,4-Dihydroxyphenyl)azo]-benzolsulfonsäure-natriumsalz (C.I. 14,270; Acid Orange 6), 4-[(2-Hydroxynaphth-1-yl)azo]-benzolsulfonsäure-natriumsalz (C.I. 15,510; Acid Orange 7), 4-[(2,4-Dihydroxy-3-[(2,4-dimethylphenyl)azo]-phenyl)azo]-benzolsulfonsäure-natriumsalz (C.I. 20,170; Acid Orange 24), 4-Hydroxy-3-[(2-methoxyphenyl)azo]-1-naphthalinsulfonsäure-natriumsalz (C.I. 14,710; Acid Red 4), 4-Hydroxy-3-[(4-sulfonaphth-1-yl)azo]-1-naphthalin-sulfonsäure-dinatriumsalz (C.I. 14,720; Acid Red No.14), 6- Hydroxy-5-[(4-sulfonaphth-1-yl)azo]-2,4-naphthalin-disulfonsäure-trinatriumsalz (C.I. 16,255; Ponceau 4R; Acid Red 18), 3-Hydroxy-4-[(4-sulfonaphth-1-yl)azo]-2,7-naphthalin-disulfonsäure-trinatriumsalz (C.I. 16,185; Acid Red 27), 8-Amino-1-hydroxy-2-(phenylazo)-3,6-naphthalin-disulfonsäure-dinatriumsalz (C.I. 17,200; Acid Red 33; Red 33), 5- (Acetylamino)-4-hydroxy-3-[(2-methylphenyl)azo]-2,7-naphthalin-disulfonsäure-dinatriumsalz (C.I. 18,065; Acid Red 35), 2-(3-Hydroxy-2,4,5,7-tetraiod-dibenzopyran-6-on-9-yl)-benzoesäure-dinatriumsalz (C.I. 45,430; Acid Red 51), N-[6-(Diethylamino)-9-(2,4-disulfophenyl)-3H-xanthen-3-yliden]-N-ethylethanammonium-hydroxid, inneres Salz, Natriumsalz (C.I. 45,100; Acid Red 52), 8-[(4-(Phenylazo)phenyl)azo]-7-naphthol-1,3-disulfonsäure-dinatriumsalz (C.I. 27,290; Acid Red 73), 2',4',5',7'-Tetrabrom-3',6'-dihydroxyspiro[isobenzofuran-1(3H),9'-[9H]xanthen]-3-on-dinatriumsalz (C.I. 45,380; Acid Red 87), 2',4',5',7'-Tetrabrom-4,5,6,7-tetrachlor-3',6'- dihydroxyspiro-[isobenzofuran-1(3H),9'[9H]xanthen]-3-on-dinatriumsalz (C.I. 45,410; Acid Red 92), 3',6'-Dihydroxy-4',5'-diiodospiro[isobenzofuran-1(3H),9'(9H)- xanthen]-3-on-dinatriumsalz (C.I. 45425; Acid Red 95), 2-Hydroxy-3-((2-hydroxynaphth-1-yl)azo)-5-nitrobenzolsulfonsäure-natriumsalz (C.I. 15,685; Acid Red 184), 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red 195), 3-Hydroxy-4-[(4-methyl-2-sulfonphenyl)azo]-2-naphthalincarbonsäure-calciumsalz (C.I. 15,850:1; Pigment Red 57:1), 3-[(2,4-Dimethyl-5-sulfophenyl)azo]-4-hydroxy-1-naphthalin-sulfonsäure-dinatriumsalz (C.I. 14,700; Food Red No. 1; Ponceau SX; FD&C Red No. 4), 1,4- Bis[(2-sulfo-4-methylphenyl)amino]-9,10-anthrachinon-dinatriumsalz (C.I. 61,570; Acid Green 25), Bis[4-(dimethylamino)phenyl]-(3,7-disulfo-2-hydroxynaphth-1-yl)carbenium-inneres Salz, Natriumsalz (C.I. 44,090; Food Green No. 4; Acid Green 50), Bis[4-(diethylamino)-phenyl](2,4-disulfophenyl)carbenium-inneres Salz, Natriumsalz (2:1) (C.I. 42,045; Food Blue No. 3; Acid Blue 1), Bis[4-(diethylamino)phenyl](5-hydroxy-2,4-disulfophenyl)-carbenium-inneres Salz, Calciumsalz (2:1) (C.I. 42,051; Acid Blue 3), N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080; Acid Blue 7), (2-Sulfophenyl)di[4-(ethyl((4-sulfophenyl)methyl)amino)phenyl]-carbenium-dinatriumsalz Betain (C.I. 42,090; Acid Blue 9; FD&C Blue No. 1), 1-Amino-4-(phenylamino)-9,10-anthrachinon-2-sulfonsäure (C.I. 62,055; Acid Blue 25), 1-Amino-4-(cyclohexylamino)-9,10-anthrachinon-2-sulfonsäure-natriumsalz (C.I. 62045; Acid Blue 62), 2-(1,3-Dihydro-3-oxo-5-sulfo-2H-indol-2-yliden)-2,3-dihydro-3-oxo-1H-indol-5- sulfonsäure-dinatriumsalz (C.I. 73,015; Acid Blue 74), 9-(2-Carboxyphenyl)-3-[(2-methylphenyl)amino]-6-[(2-methyl-4-sulfophenyl)amino]xanthylium-inneres Salz, Natriumsalz (C.I. 45,190; Acid Violet 9), 1-Hydroxy-4-[(4-methyl-2-sulfophenyl)amino]-9,10-anthrachinon-natriumsalz (C.I. 60,730; D&C Violett No. 2; Acid Violet 43), Bis[3-nitro-4-[(4-phenylamino)-3-sulfo-phenylamino]-phenyl]-sulfon (C.I. 10,410; Acid Brown 13), 5-Amino-4-hydroxy-6-[(4-nitrophenyl)-azo]-3-(phenylazo)-2,7-naphthalin-disulfonsäure-dinatriumsalz (C.I. 20,470; Acid Black 1), 3-Hydroxy-4-[(2-hydroxynaphth-1-yl)azo]-7-nitro-1-naphthalin-sulfonsäure-chromkomplex (3:2) (C.I. 15,711; Acid Black 52), 4-(Acetylamino)-5-hydroxy-6-[(7-sulfo-4-[(4-sulfophenyl)azo]naphth-1-yl)azo]-1,7-naphthalindisulfonsäure-tetranatriumsalz (C.I. 28,440; Food Black No. 1), 3',3",5',5"-Tetrabromphenolsulfonphthalein (Bromphenolblau).

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Kationische direktziehende Farbstoffe:
Als kationische direktziehende Farbstoffe eignen sich insbesondere 9-(Dimethylamino)-benzo[a]phenoxazin-7-ium-chlorid (C.I. 51,175; Basic Blue 6), Di[4-(diethylamino)phenyl][4-(ethylamino)naphthyl]carbenium-chlorid (C.I. 42,595; Basic Blue 7), Di-(4-(dimethylamino)phenyl)-(4-(methyl-phenylamino)-naphthalin-1-yl)carbenium-chlorid (C.I. 42,563; Basic Blue 8), 3,7-Di(dimethylamino)phenothiazin-5-ium-chlorid (C.I. 52,015 Basic Blue 9), Di[4-(dimethylamino)phenyl][4-(phenylamino)naphthyl] carbenium-chlorid (C.I. 44,045; Basic Blue 26), 2-[(4-(Ethyl(2-hydroxyethyl)amino)phenyl)azo]-6-methoxy-3-methyl-benzothiazolium-methylsulfat (C.I. 11,154; Basic Blue 41), 8-Amino-2-brom-5-hydroxy-4-imino-6-[(3-(trimethylammonio)phenyl)amino)-1(4H)-naphthalinon-chlorid (C.I. 56,059; Basic Blue No. 99), Bis[4- (dimethylamino)phenyl]-[4-(methylamino)phenyl]carbenium-chlorid (C.I. 42,535; Basic Violet 1), Tri(4-amino-3-methylphenyl)carbenium-chlorid (C.I. 42,520; Basic Violet 2), Tri[4-(dimethylamino)-phenyl]carbenium-chlorid (C.I. 42,555; Basic Violet 3), 2-[3,6-(Diethylamino)dibenzopyranium-9-yl]- benzoesäurechlorid (C.I. 45,170; Basic Violet 10), Di(4-aminophenyl)(4-amino-3- methylphenyl)carbeniumchlorid (C.I. 42,510 Basic Violet 14), 1,3-Bis[(2,4-diamino-5-methylphenyl)azo]-3-methylbenzol (C.I. 21,010; Basic Brown 4), 1-[(4-Aminophenyl)azo]-7-(trimethylammonio)- 2-naphthol-chlorid (C.I. 12,250; Basic Brown 16), 1-[(4-Amino-2-nitrophenyl)azo]-7-(trimethylammonio)-2-naphtholchlorid, 1-[(4-Amino-3-nitrophenyl)azo]-7-(trimethylammonio)-2-naphthol-chlorid (C.I. 12,251; Basic Brown 17), 3-[(4-Amino-2,5-dimethoxyphenyl)azo]-N,N,N-trimethylbenzolaminiumchlorid (C.I. 12,605, Basic Orange 69), 3,7-Diamino-2,8-dimethyl- 5-phenylphenazinium-chlorid (C.I. 50,240; Basic Red 2), 1,4-Dimethyl-5-[(4-(dimethylamino)phenyl)azo]-1,2,4-triazolium-chlorid (C.I. 11,055; Basic Red 22), 2-Hydroxy-1-[(2-methoxyphenyl)azo]-7-(trimethylammonio)-naphthalin-chlorid (C.I. 12,245; Basic Red 76), Di[4-(dimethylamino)phenyl]iminomethan-hydrochlorid (C.I. 41,000; Basic Yellow 2), 2-[2-((2,4-Dimethoxyphenyl)amino)ethenyl]-1,3,3-trimethyl-3H-indol-1-ium-chlorid (C.I. 48,055; Basic Yellow 11), 3-Methyl-1-phenyl-4-[(3-(trimethylammonio)phenyl)azo]-pyrazol-5-on-chlorid (C.I. 12,719; Basic Yellow 57), Bis[4-(diethylamino)phenyl]phenylcarbenium-hydrogensulfat (1:1) (C.I. 42,040; Basic Green 1), Di(4-(dimethylamino)phenyl)-phenylmethanol (C.I. 42,000; Basic Green 4), 1-(2- Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid und direktziehende Farbstoffe, die einen Heterocyclus enthalten, der mindestens ein quaternäres Stickstoffatom aufweist.

Bevorzugte kationische direktziehende Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist.

Bevorzugte kationische direktziehende Farbstoffe der Gruppe (c) sind insbesondere die folgenden Verbindungen:

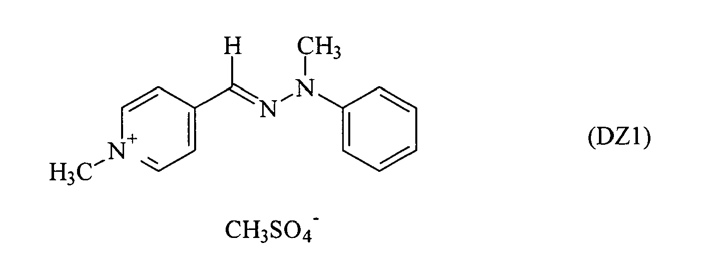

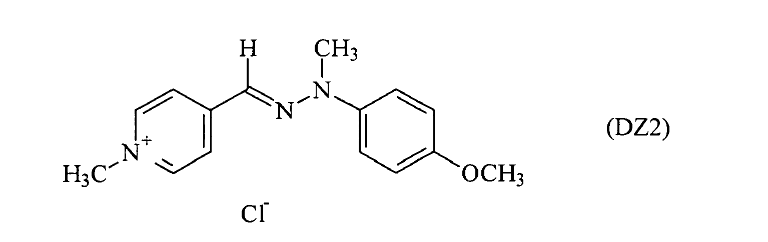

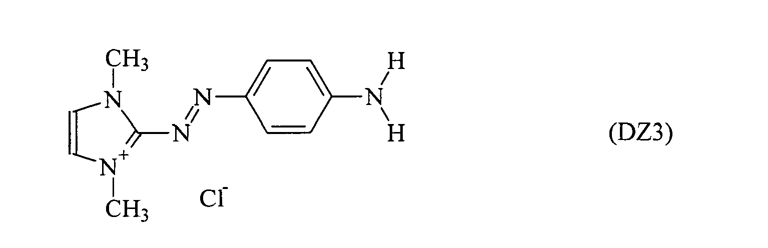

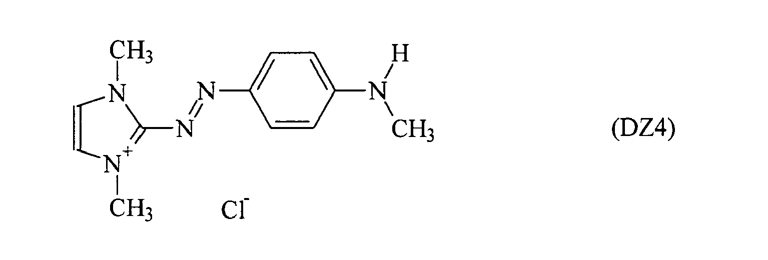

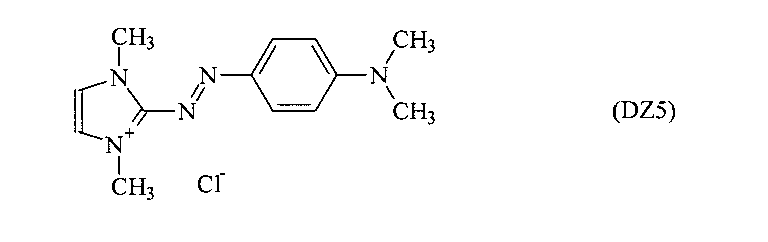

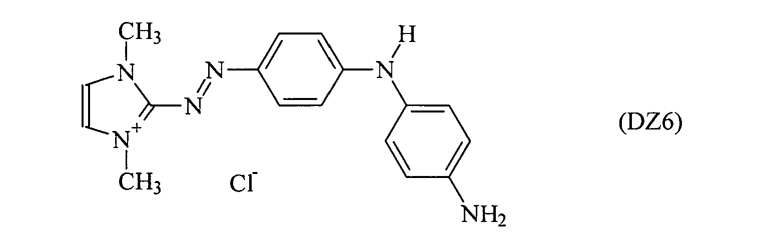

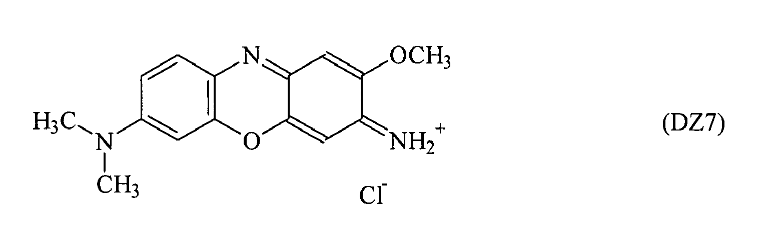

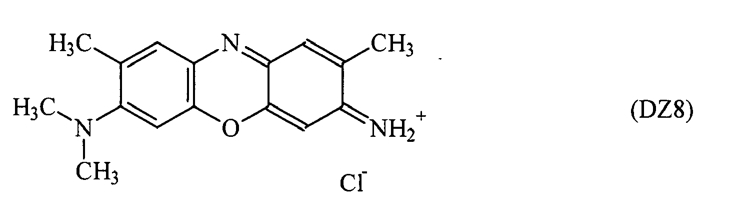

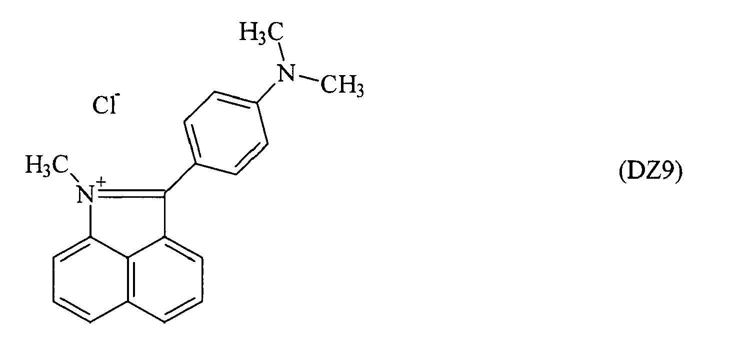

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Nichtionische direktziehende Farbstoffe:
Als nichtionische direktziehende Farbstoffe eignen sich insbesondere nichtionische Nitro- und Chinonfarbstoffe und neutrale Azofarbstoffe.

Geeignete blaue Nitrofarbstoffe sind insbesondere:
1,4-Bis[(2-hydroxyethyl)amino]-2-nitrobenzol, 1-(2-Hydroxyethyl)amino-2-nitro-4-[di(2-hydroxyethyl)-amino]-benzol (HC Blue 2), 1-Methylamino-4- [methyl-(2,3-dihydroxypropyl)amino]-2-nitrobenzol (HC Blue 6),1-[(2,3- Dihydroxypropyl)-amino]-4-[ethyl-(2-hydroxyethyl)amino]-2-nitrobenzol-hydrochlorid (HC Blue 9), 1-[(2,3-Dihydroxypropyl)amino]-4-[methyl-(2-hydroxyethyl)amino]-2-nitrobenzol (HC Blue 10), 4-[Di(2-hydroxyethyl)amino]-1-[(2-methoxyethyl)amino]-2-nitrobenzol (HC Blue 11), 4-[Ethyl-(2-hydroxyethyl)-amino]-1-[(2-hydroxyethyl)amino]-2-nitrobenzol-hydrochlorid (HC Blue 12), 2-((4-Amino-2-nitrophenyl)amino)-5-dimethylamino-benzoesäure (HC Blue 13), 1-Amino-3-methyl-4-[(2-hydroxyethyl)amino]-6-nitrobenzol (HC Violet 1), 1-(3-Hydroxypropylamino)-4-[di(2-hydroxyethyl)amino]-2-nitrobenzol (HC Violet 2), 1-(2-Aminoethylamino)-4-[di(2-hydroxyethyl)amino]-2-nitrobenzol, 4-(Di(2-hydroxyethyl)amino)-2-nitro-1-phenylamino-benzol.

Geeignete rote Nitrofarbstoffe sind insbesondere:
1-Amino-4-[(2-hydroxyethyl)amino]-2-nitrobenzol (HC Red 7), 2-Amino-4,6-dinitrophenol (Pikraminsäure) und deren Salze, 1,4-Diamino-2-nitrobenzol (C.I. 76,070), 4-Amino-2-nitro-diphenylamin (HC Red 1), 1-Amino-4-[di(2-hydroxyethyl)amino]-2-nitrobenzol-hydrochlorid (HC Red 13), 1-Amino-4-((2-hydroxyethyl)-amino]-5-chlor-2-nitrobenzol, 4-Amino-1-[(2-hydroxyethyl)amino]-2-nitrobenzol (HC Red 3), 4-[(2-Hydroxyethyl)methylamino]-1-(methylamino)-2-nitrobenzol, 1-Amino-4-[(2,3-dihydroxypropyl)amino]-5-methyl-2-nitrobenzol, 1-Amino-4-(methylamino)-2-nitrobenzol, 4-Amino-2-nitro-1-[(prop-2-en-1-yl)-amino]-benzol, 4-Amino-3-nitrophenol, 4-[(2-Hydroxyethyl)-amino]-3-nitrophenol, 4-[(2-Nitrophenyl)amino]phenol (HC Orange 1), 1-[(2-Aminoethyl)amino]-4-(2-hydroxyethoxy)-2-nitrobenzol (HC Orange 2), 4-(2,3-Dihydroxypropoxy)-1-[(2-hydroxyethyl)amino]-2-nitrobenzol (HC Orange 3), 1-Amino-5-chlor-4-[(2,3-dihydroxypropyl)amino]-2-nitrobenzol (HC Red 10), 5-Chlor-1,4-[di(2,3-dihydroxypropyl)amino]-2-nitrobenzol (HC Red 11), 2-[(2- Hydroxyethyl)amino]-4,6-dinitrophenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol (HC Red BN), 2,5-Diamino-6-nitropyridin, 6-Amino-3-[(2-hydroxyethyl)amino]-2-nitropyridin, 3-Amino-6-[(2-hydroxyethyl)amino]-2-nitropyridin, 3-Amino-6-(ethylamino)-2-nitropyridin, 3-[(2-Hydroxyethyl)amino]-6-(methylamino)-2-nitropyridin, 3- Amino-6-(methylamino)-2-nitropyridin, 6-(Ethylamino)-3-[(2-hydroxyethyl)amino]-2-nitropyridin, 1,2,3,4-Tetrahydro-6-nitrochinoxalin, 7-Amino-3,4-dihydro-6-nitro-2H-1,4-benzoxazin (HC Red 14).

Geeignete gelbe Nitrofarbstoffe sind insbesondere:
1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 4-[(2,3-Dihydroxypropyl)-amino]-3-nitro-1-trifluormethyl-benzol (HC Yellow 6), 2-[Di(2-hydroxyethyl)amino]-5-nitrophenol, 2-[(2- Hydroxyethyl)amino]-1-methoxy-5-nitrobenzol, 2-Amino-3-nitrophenol, 2-Amino-4-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluormethyl-benzol (HC Yellow 13), 4-[(2-Hydroxyethyl)-amino]-3-nitro-benzonitril (HC Yellow 14), 4-[(2-Hydroxyethyl)amino]-3-nitro-benzamid (HC Yellow 15) 3-[(2-Hydroxyethyl)amino]-4-methyl-1-nitrobenzol, 4-Chlor-3-[(2-hydroxyethyl)amino]-1-nitrobenzol.

Geeignete Chinonfarbstoffe sind insbesondere:
1,4-Di[(2,3-dihydroxypropyl)amino]-9,10-anthrachinon, 1,4-Di[(2-hydroxyethyl)amino]-9,10-anthrachinon (C.I. 61,545, Disperse Blue 23), 1-[(2-Hydroxyethyl)amino]-4-methylamino-9,10-anthrachinon (C.I. 61,505, Disperse Blue 3), 2-[(2-Aminoethyl)amino]-9,10-anthrachinon (HC Orange 5), 1-Amino-4-hydroxy-9,10-anthrachinon (C.I. 60,710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (C.I. 75,470, Natural Red 4), 1-[(3-Aminopropyl)amino]-4-methylamino-9,10-anthrachinon (HC Blue 8), 1-[(3-Aminopropyl)-amino]-9,10-anthrachinon (HC Red 8), 1,4-Diamino-2-methoxy-9,10-anthrachinon (C.I. 62,015, Disperse Red 11, Solvent Violet No. 26), 1,4-Dihydroxy-5,8-bis[(2-hydroxyethyl)amino]-9,10-anthrachinon (C.I. 62,500, Disperse Blue 7, Solvent Blue No. 69), 1,4-Diamino-9,10-anthrachinon (C.I. 61, 100, Disperse Violet 1), 1-Amino-4-(methylamino)-9,10-anthrachinon (C.I. 61, 105, Disperse Violet 4, Solvent Violet No. 12), 2-Hydroxy-3-methoxy-1,4-naphthochinon, 2,5-Dihydroxy-1,4-naphthochinon, 2-Hydroxy-3-methyl-1,4-naphthochinon, N-{6-[(3-Chlor-4-(methylamino)phenyl)imino]-4-methyl-3-oxo-1,4-cyclohexadien-1-yl}harnstoff (HC Red 9), 2-{{4-[Di(2-hydroxyethyl)amino]phenyl}amino}-5-[(2-hydroxyethyl)amino]-2,5-cyclohexadien-1,4-dion (HC Green 1), 5-Hydroxy-1,4-naphthochinon (C.I. 75,500, Natural Brown 7), 2-Hydroxy-1,4-naphthochinon (C.I. 75,480, Natural Orange 6), 1,2-Dihydro-2-(1,3-dihydro-3-oxo-2H-indol-2-yliden)-3H-indol-3-on (C.I. 73,000), 4-{{5-[(2-Hydroxyethyl) amino]-1-methyl-1 H- pyrazol-4-yl}imino}-4,5-dihydro-5-[(2-hydroxyethyl)-imino]-1-methyl-1H-Pyrazol-sulfat(1:1), Hydrat(1:1).

Geeignete neutrale Azofarbstoffe sind insbesondere:
1-[Di(2-hydroxyethyl)amino]-3-methyl-4-[(4-nitrophenyl)azo]-benzol (C.I. 11,210, Disperse Red 17), 1-[Di(2-hydroxyethyl)amino]-4-[(4-nitrophenyl)azo]-benzol (Disperse Black 9), 4-[(4-Aminophenyl)azo]-1-[di(2-hydroxyethyl)amino]-3-methylbenzol (HC Yellow 7), 2,6-Diamino-3-[(pyridin-3-yl)azo]-pyridin, 2-{[4-(Acetylamino)phenyl]azo}-4-methylphenol (C.I. 11855; Disperse Yellow 3), 4-[(4-Nitrophenyl)azo]-anilin (C.I. 11,005; Disperse Orange 3).

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Neben den direktziehenden Farbstoffen können die Haarfärbemitel weiterhin mindestens eine Vorstufe eines naturanalogen Farbstoffs enthalten. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (NAV I), 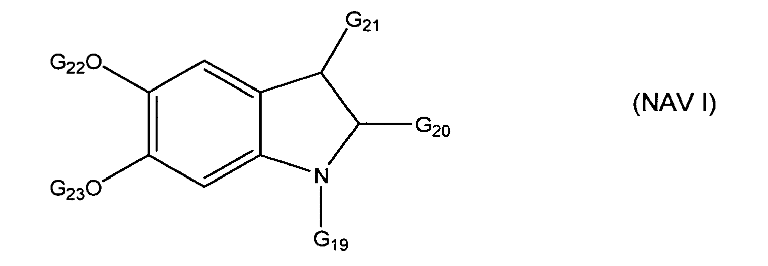 in der unabhängig voneinander
- G₁₉ steht für Wasserstoff, eine C₁-C₄-Alkylgruppe oder eine C₁-C₄-Hydroxy-alkylgruppe,
- G₂₀ steht für Wasserstoff oder eine -COOH-Gruppe, wobei die -COOH-Gruppe auch als Salz mit einem physiologisch verträglichen Kation vorliegen kann,
- G₂₁ steht für Wasserstoff oder eine C₁-C₄-Alkylgruppe,
- G₂₂ steht für Wasserstoff, eine C₁-C₄-Alkylgruppe oder eine Gruppe -CO-G₂₄, in der G₂₄ steht für eine C₁-C₄-Alkylgruppe, und
- G₂₃ steht für eine der unter G₂₂ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbon-säure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (NAV II), 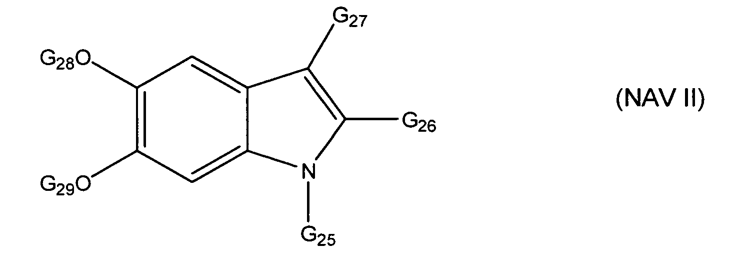 in der unabhängig voneinander
- G₂₅ steht für Wasserstoff, eine C₁-C₄-Alkylgruppe oder eine C₁-C₄-Hydroxyalkylgruppe,
- G₂₆ steht für Wasserstoff oder eine -COOH-Gruppe, wobei die -COOH-Gruppe auch als Salz mit einem physiologisch verträglichen Kation vorliegen kann,
- G₂₇ steht für Wasserstoff oder eine C₁-C₄-Alkylgruppe,
- G₂₈ steht für Wasserstoff, eine C₁-C₄-Alkylgruppe oder eine Gruppe -CO-G₃₀, in der G₃₀ steht für eine C₁-C₄-Alkylgruppe, und
- G₂₉ steht für eine der unter G₂₈ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind üblicherweise in Mengen von 0,05-10 Gew.-%, vorzugsweise 0,2-5 Gew.-% enthalten.

Die eingesetzten Haarfärbemittel enthalten die direktziehenden Farbstoffe und gegebenenfalls weitere Bestandteile in einen kosmetisch akzeptablen Träger. Dabei handelt es sich bevorzugt um ein wässriges, ein alkoholisches oder ein wässrigalkoholisches Medium mit vorzugsweise mindestens 10 Gewichtsprozent Wasser bezogen auf das gesamte Haarfärbemittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Besonders bevorzugt handelt es sich bei dem kosmetisch akzeptablen Träger um Wasser oder ein Ethanol/Wasser-Gemisch.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Haarfärbemittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gewichtsprozent.

Die eingesetzten Haarfärbemittel weisen bevorzugt einen pH-Wert von 3 bis 12 auf. Die Angaben zum pH-Wert beziehen sich dabei im Sinne dieser Schrift auf den pH-Wert bei 25°C, sofern nichts anderes vermerkt ist. Falls erforderlich können zur Einstellung des gewünschten pH-Werts übliche Alkalisierungsmittel oder Mittel zum Ansäuern der Zusammensetzung zugegeben werden.

Die eingesetzten Haarfärbemittel können weiterhin die für solche Mittel üblichen Inhaltsstoffe enthalten. Dabei ist zu berücksichtigen, dass die Haarfärbemittel auf das Haar aufgesprüht werden. Die Gesamtzubereitung des Haarfärbemittels muss daher eine Zusammensetzung und Konsistenz aufweisen, die in Form eines Aerosolsprays oder mittels sonstiger Hilfsmittel versprüht werden kann.

Die Haarfärbemittel können gegebenenfalls Tenside und/oder Emulgatoren enthalten. Menge und Art dieser Inhaltsstoffe ist so zu wählen, dass das Mittel beim Versprühen keinen Schaum bildet. Schaumbildung geht mit einer starken Volumenzunahme einher, so dass das zielgerichtete Aufbringen des Haarfärbemittels auf bestimmte Haarpartien mit genau definierten Grenzen erschwert oder gar unmöglich gemacht würde.

Als besonders vorteilhaft hat es sich erwiesen, wenn das Haarfärbemittel weiterhin mindestens einen Pflegestoff enthält. Dabei kann es sich beispielsweise um ein kationisches Tensid handeln, wobei deren Tendenz zur Förderung der Schaumbildung zu berücksichtigen ist.

Als Pflegestoffe eignen sich weiterhin Proteinhydrolysate und/oder Proteihydrolysat-Derivate, Silikonöle und/oder Silikongums, kationische Polymere, Vitamine, Provitamine, Vitaminvorstufen und/oder derer Derivate, insbesondere Panthenol, und Pflanzenextrakte. Panthenol ist ein bevorzugter Pflegestoff.

Die Haarfärbemitel können weiterhin mindestens einen UV-Filter enthalten. Durch Zugabe eines UV-Filters können sowohl die Produkte selbst, als auch die behandelten Fasern vor schädlichen Einflüssen von UV-Strahlung geschützt werden. Bevorzugt wird als UV-Filter Benzophenone-4 eingesetzt.

Weitere Wirk-, Hilfs- und Zusatzstoffe, sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Parfümöle, Dimethylisosorbid und Cyclodextrine,
- quaternierte Amine wie Methyl-1-alkylamidoethyl-2-alkylimidazolinium-methosulfat
- Farbstoffe zum Anfärben des Mittels,
- Antischuppenwirkstoffe wie Piroctone Olamine, Zink Omadine und Climbazol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wasserstoffperoxid und andere Oxidationsmittel,
- Antioxidantien.

In einer ersten bevorzugten Ausführungsform des erfindungsgemäßen Verfahrens wird das Haarfärbemittel mittels eines speziellen Sprühsystems auf das Haar aufgesprüht. Dieses System ermöglicht ein sehr präzises Arbeiten, so dass auch komplexe Muster mit geringem Aufwand erstellt werden können. Besonders einfach lassen sich mit diesem System einzelne Strähnen einfärben.

Das Sprühsystem weist eine Düse, mindestens eine Produktzufuhr, eine Gaszufuhr, eine Steuereinrichtung für die Gaszufuhr und im festen Abstand d vor der Düse ein Selektionselement auf. Das Selektionselement dient dazu, einzelne Haarsträhnen zu selektieren und den Abstand zwischen Haarsträhne und Düse während des Colorationsvorgangs konstant zu halten.

Um Haarsträhnen zu färben, kämmen Friseure üblicherweise einen Scheitel und weben, beispielsweise mit der spitzen Ende eines Kammes oder eines Färbepinsels, eine einzelne Haarsträhne aus der durch den Scheitel definierten Haarfläche heraus. In einem nächsten Schritt wird unter diese heraus getrennte Haarsträhne eine Aluminiumfolie gelegt. Diese trennt die selektierte Haarsträhne vom Rest der Haare.

Selbstverständlich lässt sich diese Vorgehensweise für weitere Haarsträhnen wiederholen. Die auf der Aluminiumfolie liegenden Haarsträhnen können nun mit dem Haarbehandlungsmittel behandelt werden. Anschließend werden die behandelten Haarsträhnen mit dem Rest der Aluminiumfolie zugedeckt und somit vollständig eingepackt.

Durch Aufsprühen des Haarfärbemittels mittels oben genannten speziellen Sprühsystems vereinfacht sich dieses Vorgehen um ein Vielfaches. So kann der Verwender dieses Sprühsystems in gleicher Weise durch Kämmen eines Scheitels eine Haarfläche definieren, jedoch anstelle des Webens wird eine einzelne Haarsträhne durch ein einfaches Durchstechen und Zurückziehen der Haarfläche mit dem ringförmigen Selektionselement erzielt. Aufgrund des definierten Abstandes zwischen Düse und der durch das Selektionselement gehaltenen Haarsträhne ist eine Aluminiumfolie wie im Stand der Technik zum Schutz der restlichen Haare nicht notwendig. Vielmehr kann in einfacher Weise durch Ziehen des Sprühsystems bei gleichzeitiger Applikation des Produkts die Haarsträhne in einem einzigen zügigen Arbeitsschritt behandelt werden. Dieser Vorgang ist je nach Anzahl der Haarsträhnen beliebig oft zu wiederholen.

Im Gegensatz zu bekannten Colorationsmethoden für Haare sind wesentlich weniger Hilfsmittel erforderlich. Anstelle eines Kammes, einer Färbeschale, eines Pinsels und der Aluminiumsfolien sind nur der Kamm und das genannte Sprühsystem notwendig. Dies ermöglicht ein deutlich einfacheres und zügigeres Arbeiten, da bei nur zwei benötigten Geräten, keines aus der Hand des Verwenders gelegt werden muss. Auch der Verzicht auf Aluminiumfolien beschleunigt dieses Behandlungsverfahren. Ebenfalls nicht zu vernachlässigen ist der monetäre und ökologische Effekt durch den Verzicht auf Aluminiumfolie.

Ein weiterer Vorteil dieser direkten Applikation ist die Einsparung von Produkt. Da nun in exakter und gezielter Weise Produkt auf einzelne Haarsträhnen appliziert werden kann, benötigt man bei der Verwendung eines erfindungsgemäßen Sprühsystems wesentlich weniger Produkt als bisher. Über die variable Produktabgabe, welche über die Variation der Gaszufuhr geregelt wird, können sogar in einfacher Weise Farb- oder Produktverläufe über den gesamten Verlauf der Haarsträhne realisiert werden.

Ein weiterer Vorteil ist die einfache Handhabung. Durch den vordefinierten Abstand d zwischen Haarsträhnen und Düse ist es auch für Laien und Anfänger möglich, Haarsträhnen zu selektieren und Produkt in geeigneter Weise zu applizieren. Darüber hinaus sind Benutzer, die mit dem Umgang des Sprühsystems vertraut sind, in der Lage durch Variation der Gaszufuhr völlig neuartige Behandlungen und Ergebnisse zu erzielen. Beispielsweise ist das Colorieren von einer einzelnen Haarsträhne mit unterschiedlichen Farben mit herkömmlichen Sprühsystemen überhaupt nicht durchführbar.

Es ist bevorzugt, wenn das Selektionselement abnehmbar gestaltet ist. Dies ist insbesondere zu empfehlen, um die Möglichkeit zur Reinigung des Selektionselementes zu geben. Auch können so unterschiedliche Selektionselemente an das Sprühsystem angebracht werden, wodurch die Variationsmöglichkeiten der Haarsträhnenbehandlung, beispielsweise durch unterschiedliche Breiten oder Formen des Selektionselementes noch weiter vergrößert wird.

Eine weitere Möglichkeit die Variabilität weiter zu steigern ist es, dass das Selektionselement verschiebbar angebracht ist. So kann der Benutzer des Sprühsystems den Abstand zwischen Düse und Selektionselement an die von ihm zu bearbeitenden Haarsträhne anpassen.

Ebenfalls kann es sinnvoll sein, das Selektionselement derart zu gestalten, dass die Breite a der Öffnung des Selektionselementes variabel einstellbar ist. Diese Breite a ist verantwortlich für die Anzahl von Haaren, welche beim Selektionsvorgang im Haken gehalten werden. Durch die Variation dieser Breite kann gesteuert werden, welche Anzahl an Haaren und damit, welche Breite der Haarsträhnen selektiert wird. Es verbleibt also eine Vielzahl von Variationsmöglichkeiten für den Benutzer, ohne die Einfachheit der Applikationsmöglichkeit zu beeinträchtigen.

Um ein unerwünschtes Anfärben benachbarter Haarregionen sicher zu vermeiden, kann es vorteilhaft sein, ein Sprühsystem einzusetzen, dessen Selektionselement einen Spritzschutz aufweist.

Insbesondere zum Fäerben einzelner Strähnen wird vorzugsweise ein Sprühsystem eingesetzt, dessen Selektionselement als Haken ausgestaltet ist, der zumindest teilweise einen runden Querschnitt aufweist.

Besonders vorteilhaft ist ein Sprühsystem, das eine oder mehrere Vorratskammern für ein oder mehrere Haarfärbemittel aufweist. Über eine entsprechende Anzahl Produktzufuhren können so unterschiedliche Farben auf einer Haarsträhne auf einfachste Art und Weise realisiert werden.

Um eine besonders hohe Flexibilität zu gewährleisten, kann es sinnvoll, sein die Steuereinrichtung der Gaszufuhr stufenlos einstellbar zu realisieren. Dies erfordert zwar mehr Fingerspitzengefühl, erhöht jedoch die Flexibilität.

Die Realisierung derartiger stufenloser Steuereinrichtungen kann beispielsweise einfach durch eine axial bewegliche Nadel innerhalb des Konus der Düse gestaltet werden. Selbstverständlich sind auch andere Lösungen für eine stufenlose Regelung denkbar.

Bei Einsatz des genannten Sprühsystems wird das Haarfärbemittel mittels eines Gases, das über die Gaszufuhr in das Sprühsystem eingetragen wird, auf das Haar aufgesprüht. Das Sprühsystem lässt sich problemlos mit einem Gas betreiben, welches einen hohen Sauerstoffanteil aufweist. Bevorzugt wird ein Sauerstoffanteil über 30%, besonders bevorzugt ein Sauerstoffanteil über 50%, ganz besonders bevorzugt kann auch nahezu technisch reiner Sauerstoff verwendet werden.

Ein geeignetes Sprühsystem, das gemäß der ersten bevorzugten Ausführungsform des erfindungsgemäßen Verfahrens eingesetzt wird, wird anhand der Zeichnung mit den Figuren 1 bis 2 im Folgenden näher beschrieben.

### Darin zeigt

- Fig. 1:: Eine schematische Darstellung des Sprühsystems;
- Fig. 2:: Eine Ausführungsform eines Selektionselementes;

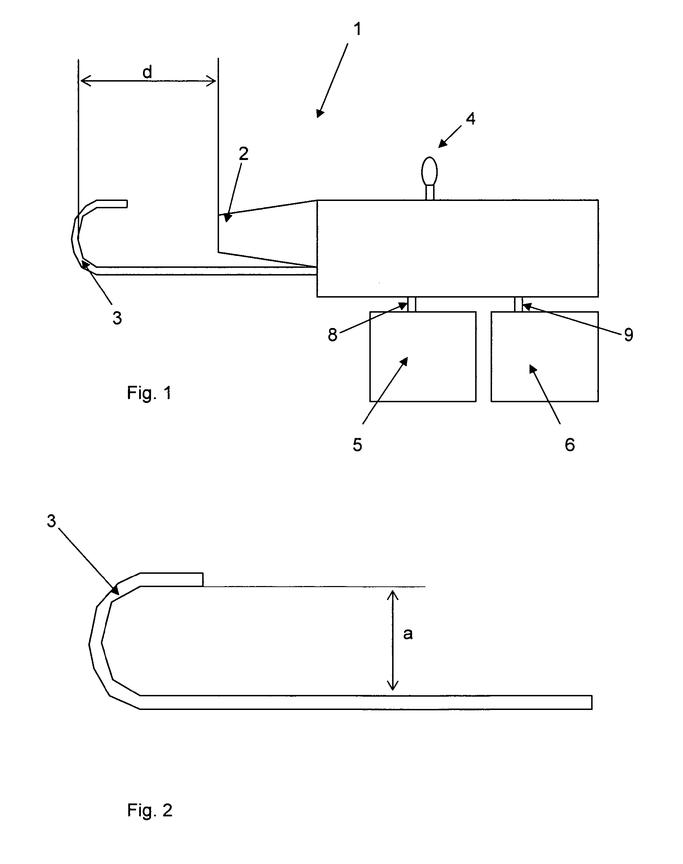

Bei der in Figur 1 dargestellten Ausführungsform des Sprühsystems 1 sind an der Unterseite ein Produktbehälter 5 und ein Gasbehälter 6 angebracht, die mittels Produktzufuhr 8 bzw. Gaszufuhr 9 mit dem Sprühsystem verbunden sind. An der Oberseite des Sprühsystems 1 ist eine Steuereinrichtung 4 in Form eines Hebels dargestellt. In festem Abstand d von der Düse 2 ist ein Selektionselement 3 dargestellt.

Figur 2 ist die vergrößerte Darstellung des Selektionselements 3 aus Figur 1. Die für den Selektionsvorgang wesentliche Breite der Öffnung ist mit dem Buchstaben a gekennzeichnet.

Das in der Zeichnung dargestellte Sprühsystem 1 wird zur Haarbehandlung vorzugsweise wie folgt verwendet:
Der Benutzer hält das einsatzbereite Sprühsystem 1 in der einen und einen Kamm in der anderen Hand. Mit dem Kamm kämmt er die Haare zu einem Scheitel und erhält somit eine definierte Haarfläche. Mit dem Selektionselement 3 durchsticht er die Haarfläche. Beim Zurückziehen verbleibt ein definierter Teil der Haare bzw. eine definierte Haarsträhne innerhalb des Hakens. Vorzugsweise findet dieser Vorgang so nahe wie möglich am Haaransatz statt. Die so selektierte Haarsträhne kann nun behandelt werden. Die Behandlung findet statt, indem einerseits gleichzeitig das Sprühsystem 1 vom Haaransatz weg gezogen wird und somit die Haarsträhne innerhalb des Selektionselementes 3 entlang rutscht und andererseits über die Steuereinrichtung 4 die Produktabgabe geregelt wird. Erreicht der Benutzer das Ende der Haarsträhne, so beendet er mit der Steuereinrichtung 4 die Produktabgabe und die Haarsträhne rutscht aus dem Selektionselement 3 heraus und wird somit freigegeben. Aufgrund der bereits erfolgten Trocknung des Produktes auf der Haarsträhne, stellt das Zurückfallen der behandelten Haarsträhne in das Resthaar kein Problem dar. Der Benutzer kann nun entweder in der noch vorliegenden definierten Haarfläche die nächste Haarsträhne in gleicher Weise behandeln oder durch neues Kämmen eine neue Fläche für eine neue Haarsträhne definieren.

Diese beschriebene Vorgehensweise kann der Benutzer selbstverständlich frei variieren. So können beim Colorieren nur einzelne Teile der Haarsträhne behandelt werden oder einzelne Teile unterschiedlich intensiv. Auf diese Weise lassen sich neben einfachen Strähnen auch komplexere graphische Muster erzielen.

In einer weiteren bevorzugten Ausführungsform des erfindungsgemäßen Verfahrens enthält das Haarfärbemittel bereits ein Treibmittel und ist in einer Aerosoldose konfektioniert. Aus der Aerosoldose wird das Haarfärbemittel durch Betätigen des Ventils direkt auf die gewünschte Haarpartie aufgesprüht. Vorteilhaft an diesem Vorgehen ist, dass keine spezielle Applikationsvorrichtung benötigt wird. Allerdings ist die Flexibilität etwas eingeschränkt, da eine Aerosoldose nur ein Haarfärbemittel enthält, so dass zum Aufbringen verschiedener Farben mehrere Aerosoldosen zum Einsatz kommen. Die Größe des Sprühkegels bestimmt die Genauigkeit, mit der einzelne Konturen auf das Haar aufgebracht werden können, und lässt sich durch Wahl des Ventils, sowie den Abstand von Ventilauslass zu dem zu behandelnden Haar einstellen.

Als Treibmittel können alle in der Kosmetik üblicherweise verwendeten Treibmittel zum Einsatz kommen. Geeignete Treibmittel sind beispielsweise N₂O, Dimethylether, CO₂, Luft und Alkane mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und deren Mischungen.

Bei Verwendung herkömmlicher Sprühvorrichtungen enthalten die Haarfärbemittel das Treibmittel bevorzugt in Mengen von 1 bis 80 Gew.-%, bezogen auf das gesamte Produkt. Mengen von 5 bis 70 Gew.-%, insbesondere von 10 bis 70 Gew.-%, sind besonders bevorzugt.

Im Rahmen des erfindungsgemäßen Verfahrens kann das Haarfärbemittel nach dem Aufsprühen und einer gewissen Einwirkzeit wieder aus dem Haar ausgespült werden. Man spricht von sogenannten rinseoff-Produkten. Es ist aber auch möglich, dass die Haarfärbemittel nach dem Aufbringen im Haar verbleiben, es sich bei den Haarfärbemitteln also um sogenannte leave-on-Produkte handelt.

## Patentansprüche

1. Verfahren zur Erzeugung graphischer Muster auf menschlichem Haar, **dadurch gekennzeichnet, dass** ein Haarfärbemittel, enthaltend mindestens einen direktziehenden Farbstoff in einem kosmetisch akzeptablen Träger in dem gewünschten Muster auf das zu behandelnde Haar aufgesprüht wird.

2. Verfahren nach Anspruch 1, **dadurch gekennzeichnet, dass** der direktziehende Farbstoff ausgewählt ist aus Nitrophenylendiaminen, Nitroaminophenolen, Azofarbstoffen, Anthrachinonen und Indophenolen.

3. Verfahren nach mindestens einem der Ansprüche 1 bis 2, **dadurch gekennzeichnet, dass** das Haarfärbemittel mittels eines Sprühsystems (1) aufgesprüht wird, wobei das Sprühsystems (1) eine Düse (2), mindestens eine Produktzufuhr (8), eine Gaszufuhr (9), eine Steuereinrichtung (4) für die Gaszufuhr (9) und im festen Abstand d vor der Düse (2) ein Selektionselement (3) aufweist.

4. Verfahren nach Anspruch 3, **dadurch gekennzeichnet, dass** das Selektionselement (3) abnehmbar am Sprühsystem (1) angebracht ist.

5. Verfahren nach mindestens einem der Ansprüche 3 bis 4, **dadurch gekennzeichnet, dass** das Selektionselement (3) verschiebbar angebracht ist.

6. Verfahren nach mindestens einem der Ansprüche 3 bis 5, **dadurch gekennzeichnet, dass** die Breite a einer Öffnung des Selektionselements (3) variabel fixierbar ist.

7. Verfahren nach mindestens einem der Ansprüche 3 bis 6, **dadurch gekennzeichnet, dass** das Selektionselement (3) einen Spritzschutz aufweist.

8. Verfahren nach mindestens einem der Ansprüche 3 bis 7, **dadurch gekennzeichnet, dass** das Selektionselement (3) ein Haken ist und zumindest teilweise einen runden Querschnitt aufweist.

9. Verfahren nach mindestens einem der Ansprüche 1 bis 2, **dadurch gekennzeichnet, dass** das Haarfärbemittel ein Treibmittel enthält und in einer Aerosoldose konfektioniert ist.
